(19) **Europäisches Patentamt / European Patent Office / Office européen des brevets**

(11) **EP 4 712 095 A1**

(12) # EUROPEAN PATENT APPLICATION
published in accordance with Art. 153(4) EPC

(43) Date of publication:
**18.03.2026 Bulletin 2026/12**

(21) Application number: **24806349.7**

(22) Date of filing: **29.04.2024**

(51) International Patent Classification (IPC):
*G16H 30/20* (2018.01)     *G16H 30/40* (2018.01)
*G06T 7/00* (2017.01)       *G06T 7/10* (2017.01)
*H04N 5/265* (2006.01)

(52) Cooperative Patent Classification (CPC):
Y02D 10/00

(86) International application number:
**PCT/CN2024/090491**

(87) International publication number:
**WO 2024/234993 (21.11.2024 Gazette 2024/47)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.05.2023 CN 202310536781**

(71) Applicant: **Tianjin Yujin Artificial Intelligence
Medical
Technology Co., Ltd
Tianjin 300457 (CN)**

(72) Inventors:
• **FENG, Yue**
  **Tianjin 300457 (CN)**
• **MU, Jinbao**
  **Tianjin 300457 (CN)**

(74) Representative: **Cabinet Chaillot
16/20, avenue de l'Agent Sarre
B.P. 74
92703 Colombes Cedex (FR)**

(54) **INTELLIGENT MEDICAL DISPLAY SYSTEM**

(57)     The present invention relates to an intelligent medical display system, which comprises a video acquisition module, an AI edge computing module, a marking layer output module, a video superimposition module, and a display module. The video acquisition module receives a video input signal and transmits same to the AI edge computing module; the AI edge computing module analyzes the video input signal to obtain a lesion target detection packet, a lesion segmentation packet, and a semantic scene detection packet, and pushes same to the marking layer output module; the marking layer output module groups and marks the pushed packets, performs marking on background images according to a grouping and marking result to form a lesion target detection result video, a lesion segmentation result video, and a semantic scene detection result video, and outputs same to the video superimposition module; the video superimposition module is used for performing video superimposition; the display module is used for displaying. The present invention can improve image quality and system stability, reduce image processing delay, and improve real-time performance and accuracy in a surgical process.

FIG. 1

# EP 4 712 095 A1

**Description**

CROSS-REFERENCE TO RELATED APPLICATIONS

The present invention claims the priority to Chinese Patent Application No. 202310536781.5 filed with the Chinese Patent Office on May 12, 2023 and entitled "Intelligent Medical Display System", which is incorporated herein in its entirety by reference.

TECHNICAL FIELD

The present invention belongs to the technical field of medical devices, and relates to a medical display system, and in particular, to an intelligent medical display system.

BACKGROUND OF THE INVENTION

With the rapid development of medical imaging technology and information technology, there are increasing artificial intelligence-assisted diagnosis devices on the market. Most of them directly output video signals of medical imaging devices to an artificial intelligence (AI) edge computation device, and then images are analyzed in an independent device and results are displayed. However, in the prior art, the image processing speed, stability, and versatility are limited to some extent.

Specifically, in the prior art, an independent AI edge computation device is used to analyze the video signal in real time, and output an analyzed image to a medical display or an independent display screen and is placed side by side with a display displaying an original image. Although the solution can implement real-time image analysis, it still has the following defects:

1. A single display function: the existing display can merely display original video signals or signals after AI edge processing, and cannot switch between many display modes.

2. Image quality degradation: the quality of the original image processed by the AI edge computation device is likely to degrade.

3. System stability and delay: due to the influence of an operating system and AI processing, the system may become unstable and delayed, affecting a surgical process.

4. It is impossible to catch primary attention of a doctor, leading to loss or ineffective reception of prompt information. In addition, it is impossible to implement synchronous overlaying and real-time selection and switching of multimodal prompt data after multi-channel algorithm processing.

In view of that, it is necessary to develop a novel intelligent medical display system for solving the defects in the prior art.

BRIEF SUMMARY OF THE INVENTION

In order to overcome the defects of the prior art, the present invention provides an intelligent medical display system, which can improve image quality and system stability, reduce image processing delay, and improve real-time performance and accuracy during an operation.

In order to achieve the above objective, the present invention provides the following technical solution:

an intelligent medical display system includes a video acquisition module, an artificial intelligence (AI) edge computation module, a marking layer output module, a video overlaying module, and a display module, where

the video acquisition module is configured to receive a video input signal from a medical imaging device and transmit the video input signal to the AI edge computation module;

the AI edge computation module is configured to analyze the video input signal to obtain and push a lesion target detection message, a lesion segmentation message, and a semantic scene detection message to the marking layer output module;

the marking layer output module includes an information processing submodule and an image pushing submodule, the information processing submodule is configured for packet marking on the messages pushed by the AI edge computation module, and divide the messages into a lesion target detection message packet, a lesion segmentation message packet, and a semantic scene detection message packet, and the image pushing submodule is configured to mark a background image according to a packet marking result of the information processing submodule, to form and output a lesion target detection result video, a lesion segmentation result video, and a semantic scene detection result video to the video overlaying module;

the video overlaying module is configured to overlay the lesion target detection result video, the lesion segmentation result video, and the semantic scene detection result video from the image pushing submodule and the video input signal from the video acquisition module and output an overlaid video to the display module; and

the display module is configured to display the overlaid video.

Preferably, the information processing submodule includes a first buffer and a lesion target detection buffer, a lesion segmentation buffer, and a semantic scene detection buffer that are connected to the first buffer; the first buffer is connected to the AI edge computation module to receive and temporarily store the message pushed by the AI edge computation module and parse a message header of the message; and according to the message header, the lesion target detection message is temporarily stored in the lesion target detection buffer, the lesion segmentation message is temporarily stored in the lesion segmentation buffer, and the semantic scene detection message is temporarily stored in the semantic scene detection buffer, so as to be divided into the lesion target detection message packet, the lesion segmentation message packet, and the semantic scene detection message packet respectively.

Preferably, the image pushing submodule includes a texture channel creation unit, a texture channel context creation unit, a background image drawing unit, a graphic drawing unit, and a video output unit;

the texture channel creation unit is configured to create and bind a lesion target detection texture channel, a lesion segmentation texture channel, and a semantic scene detection texture channel to three output ports respectively; the texture channel context creation unit is configured to create a lesion target detection texture channel context, a lesion segmentation texture channel context, and a semantic scene detection texture channel context for the three output ports respectively;

the background image drawing unit is configured to draw background images in the lesion target detection texture channel, the lesion segmentation texture channel, and the semantic scene detection texture channel respectively; the graphic drawing unit draws a lesion target graphic on the background image in the lesion target detection texture channel based on the lesion target detection message packet, draws a lesion segmentation graphic on the background image in the lesion segmentation texture channel based on the lesion segmentation message packet, and draws a semantic scene graphic on the background image in the semantic scene detection texture channel based on the semantic scene detection message packet; and

the video output unit is configured to form the background image and the lesion target graphic drawn thereon into the lesion target detection result video, form the background image and the lesion segmentation graphic drawn thereon into the lesion segmentation result video, and form the background image and the semantic scene detection graphic drawn thereon into the semantic scene detection result video, and output such videos to the video overlaying module through the three output ports respectively.

Preferably, the video overlaying module includes four input terminals and one output terminal, the four input terminals are configured to receive the lesion target detection result video, the lesion segmentation result video, and the semantic scene detection result video that are from the video output unit and the video input signal from the video acquisition module, and the output terminal is configured to output the overlaid video to the display module.

Preferably, the video overlaying module performs video overlaying through the following steps:

1) creating and initializing a video input signal ring buffer, a lesion detection result video ring buffer, a lesion segmentation result video ring buffer, and a semantic scene detection result video ring buffer, and setting a capacity of each ring buffer;

2) creating four listening threads of input channels of the input terminals, where when the input terminals receive a video input, the input terminals write frame data to corresponding ring buffers respectively, and when the ring buffer is full, header frame data are popped while new frame data are inserted;

3) creating a misaligned output, that is, directly outputting, when the video input signal ring buffer reaches a preset storage length for first time, first frame data without any processing, where the first frame data in the video input signal ring buffer and first frame data in the other three ring buffers are misaligned by time of a frame; and

4) outputting an overlaid and synthesized AI frame.

Preferably, step 4) specifically includes:

4.1) using output time of the first frame data of the video input signal occupied by the misaligned output for graphic overlaying on header first frame data of the lesion detection result video ring buffer, header first frame data of the lesion segmentation result video ring buffer, and header first frame data of the semantic scene detection result video ring buffer, and obtaining a synthesized AI graphic prompt signal; and

4.2) overlaying the synthesized AI graphic prompt signal and the frame data of the video input signal and obtaining the overlaid and synthesized AI frame.

Preferably, the AI edge computation module includes a lesion target detection submodule, a lesion segmentation submodule, and a semantic scene detection submodule, the lesion target detection submodule is configured to recognize the video input signal frame by frame to obtain a lesion detection object target, and convert the lesion detection object target into a message to obtain the lesion target detection message, the lesion segmentation submodule is configured to recognize the video input signal frame by frame to obtain a lesion segmentation object target, and convert the lesion segmentation object target into a message to obtain the lesion segmentation message, and the semantic scene detection submodule is configured to recognize the video input signal frame by frame to obtain a semantic scene detection result, and convert the semantic scene detection result into a message to obtain the semantic scene detection message.

Preferably, the lesion target detection message includes a message header and a message body, the message header is DET, the message body includes coordinates of a plurality of lesion detection object targets, and a plurality of coordinates are separated by a symbol "|"; the lesion segmentation message includes a message header and a message body, the message header is SEG, the message body is a set of point coordinates of a plurality of lesion segmentation object targets, and a plurality of point coordinate sets are separated by a symbol |; and the semantic scene detection message includes a message header and a message body, the message header is TXT, the message body includes prompt words corresponding to a plurality of semantic scenes, and a plurality of prompt words are separated by a symbol |.

Preferably, the display module has an image input source switch button, the video acquisition module, the AI edge computation module, and the video overlaying module are all connected to the display module, and the image input source switch button is configured to determine whether a content displayed by the display module is from the video acquisition module, the AI edge computation module, or the video overlaying module.

Compared with the prior art, the intelligent medical display system of the present invention has one or more of the following beneficial technical effects:

1. The present invention provides three display modes, such that the system has a multifunctional effect, and provides more convenience for medical personnel.

2. According to the present invention, through the AI prompt signal, the radiomics combined application and clinical technical practice of multi-modal diagnosis, graded diagnosis, and disease type diagnosis can be supported, so as to help doctors to improve quality of the medical operation.

3. Through the video overlaying technology, the present invention implements overlaid display of the original video input signal and a marked video output by the AI edge computation module, and improves image quality and system stability.

4. The present invention reduces the image processing delay, and improves the real-time performance and the accuracy during the operation.

BRIEF DESCRIPTION OF THE SEVERAL VIEWS OF THE DRAWINGS

FIG. 1 is a schematic compositional diagram of an intelligent medical display system of the present invention.
FIG. 2 is a compositional diagram and work flowchart of a marking layer output module of the present invention.
FIG. 3 is a schematic compositional diagram of an image pushing submodule of the present invention.
FIG. 4 is a flowchart of video overlaying of a video overlaying module of the present invention.

DETAILED DESCRIPTION OF THE INVENTION

The present invention will be further described below with reference to accompanying drawings and in conjunction with examples, and contents of the examples should not be taken as limitation to the protection scope of the present invention.

In order to solve the shortcomings of a medical display system in the prior art, the present invention provides an intelligent medical display system that can improve image quality and system stability, reduce image processing delay, and improve real-time performance and accuracy during an operation.

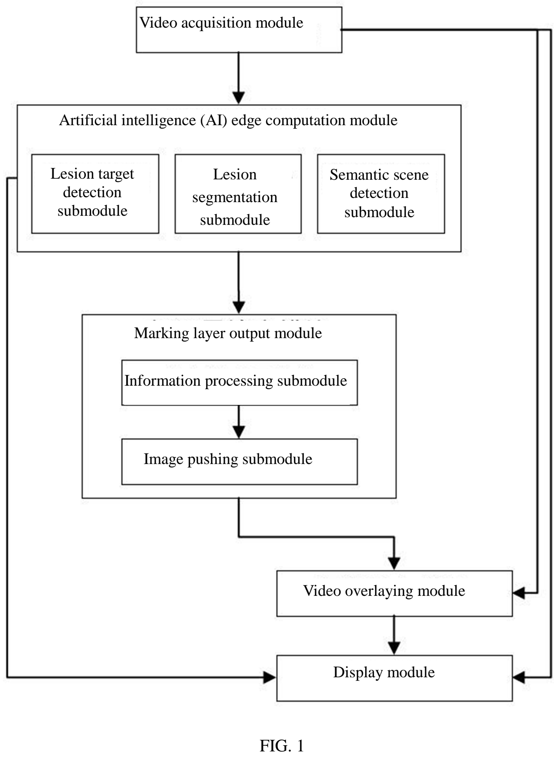

FIG. 1 shows a schematic compositional diagram of the intelligent medical display system of the present invention. As shown in FIG. 1, the intelligent medical display system of the present invention includes a video acquisition module, an artificial intelligence (AI) edge computation module, a marking layer output module, a video overlaying module, and a display module.

The video acquisition module is configured to receive a video input signal from a medical imaging device and transmit the video input signal to the AI edge computation module.

Specifically, the video acquisition module may receive the video input signals from various medical imaging devices, such as an endoscope, ultrasound, or a laparoscope. Moreover, the video acquisition module may transmit a received video input signal to the AI edge computation module through a high-speed peripheral component interface express (PCIE) interface.

The AI edge computation module is configured to analyze the video input signal to obtain and push a lesion target detection message, a lesion segmentation message, and a semantic scene detection message to the marking layer output module.

Specifically, the AI edge computation module includes a lesion target detection submodule, a lesion segmentation submodule, and a semantic scene detection submodule.

The lesion target detection submodule may be formed based on target detection algorithms such as yolov5, yolov7, and faster-rcnn. The creation steps of the lesion target detection submodule are as follows:

1. A medical image including a lesion is acquired, and the medical image is marked with a rectangular box to obtain a lesion target detection data set.
2. A lesion target detection neural network is created.

According to the present invention, the yolov5 target detection network is adopted to create the lesion target detection neural network. The lesion target detection neural network includes three cross-stage residual network structures for feature extraction. One feature pyramid scaling structure is used for feature extraction and multi-scale feature image generation, one feature pyramid aggregation structure is used for feature extraction and multi-scale feature image fusion, and one target detection head structure is used for reasoning a target defining position.

3. The lesion target detection data set is input into the lesion target detection neural network, and a lesion target detection algorithm, that is, the lesion target detection submodule is obtained through backpropagation training.

When in use, the lesion target detection submodule is used to recognize the video input signal frame by frame to obtain a lesion detection object target, and convert the lesion detection object target into a message to obtain the lesion target detection message.

The lesion target detection message includes a message header and a message body. The message header is DET, the message body includes coordinates of a plurality of lesion detection object targets, and a plurality of coordinates are separated by a symbol "|".

In the present invention, since the detection target is marked with a rectangular box, the lesion detection object target is the rectangular box. The message body includes coordinates of an upper left vertex and a lower right vertex of the rectangular box, and the coordinates of the two vertices are separated by the symbol "|".

Finally, the lesion target detection submodule pushes the lesion target detection message to the marking layer output module by using Socket technology.

The lesion segmentation submodule may be created based on image segmentation algorithms such as UNet, FCN, and Deeplab. The creation steps of the lesion segmentation submodule are as follows:

1. A medical image including a lesion is acquired, and the medical image is marked with a contour line to obtain a lesion segmentation data set.
2. A lesion segmentation neural network is created.

The present invention adopts UNet segmentation network to create the lesion segmentation neural network. The lesion segmentation neural network includes an encoder modules that consists of four convolution blocks with decreasing scale and is configured for feature extraction and outputting four feature images, and a decoder structure that consists of four convolution blocks with increasing scale and is configured for feature fusion and recreation of segmentation results.

3. The lesion segmentation data set is input into the lesion segmentation neural network, and a lesion segmentation algorithm, that is, the lesion segmentation submodule is obtained through backpropagation training.

When in use, the lesion segmentation submodule is used to recognize the video input signal frame by frame to obtain a lesion segmentation object target, and convert the lesion segmentation object target into a message to obtain the lesion segmentation message.

The lesion segmentation message includes a message header and a message body, the message header is SEG, the message body is a set of point coordinates of a plurality of lesion segmentation object targets, and a plurality of point coordinate sets are separated by a symbol |.

In the present invention, since the lesion segmentation is performed with a polygon, the lesion segmentation object target is the polygon. The message body includes coordinates of the vertices of the polygon, and the coordinates between the vertices are separated by the symbol "|".

Finally, the lesion segmentation submodule pushes the lesion segmentation message to the marking layer output module by using Socket technology.

The semantic scene detection submodule may be created based on semantic scene detection algorithms such as TSN, SlowFast, and X3D. The creation steps of the semantic scene detection submodule are as follows:

1. A medical video clip is acquired, and the medical video clip is marked with a label to obtain a semantic scene data set. In this example, the labeling marking is, for example, an endoscope liquid stool clip corresponding to a label "Pay attention to flushing the intestine", a ulcer lesion observation clip corresponding to a label "Turn on the staining light for observation, and take biopsy from the inside of the ulcer dike". It should be understood that the example is merely illustrative and includes, but is not limited to, the clips and labels.

2. A semantic scene detection neural network is created.

The present invention adopts topological scene map (TSM) semantic scene detection network to create the semantic scene detection neural network. The semantic scene detection neural network includes a convolutional neural network with 16 residual structures and configured to receive an input video clip and extract features frame by frame to obtain a frame-by-frame feature map; a spatio-temporal fusion module configured for misaligned fusion on the feature maps obtained frame by frame, that is, move a front one third of the frame-by-frame feature map backwards by one frame, move a last one third of the frame-by-frame feature map forwards by one frame, produce misaligned effect on the frame-by-frame feature map, and finally obtain the spatio-temporal feature map through fusion; a fully connected layer module configured to predict a semantic category label of the fused spatio-temporal feature map.

3. The semantic scene data set is input into the semantic scene detection neural network, and a semantic scene detection algorithm, that is, the semantic scene detection submodule is obtained through backpropagation training.

When in use, the semantic scene detection submodule is configured to recognize the video input signal frame by frame to obtain a semantic scene detection result, and convert the semantic scene detection result into a message to obtain the semantic scene detection message.

The semantic scene detection message includes a message header and a message body, the message header is TXT, the message body includes prompt words corresponding to a plurality of semantic scenes, and a plurality of prompt words are separated by a symbol |.

Finally, the semantic scene detection submodule pushes the semantic scene detection message to the marking layer output module by using Socket technology.

The marking layer output module includes an information processing submodule and an image pushing submodule.

The information processing submodule is configured for packet marking on the messages pushed by the AI edge computation module, and divide the messages into a lesion target detection message packet, a lesion segmentation message packet, and a semantic scene detection message packet. The image pushing submodule is configured to mark a background image according to a packet marking result of the information processing submodule, to form and output a lesion target detection result video, a lesion segmentation result video, and a semantic scene detection result video to the video overlaying module.

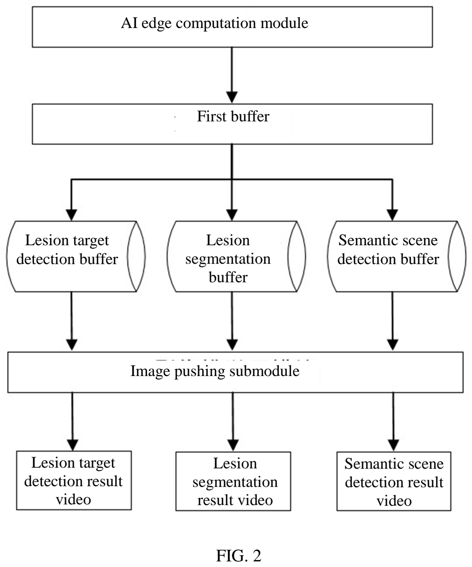

Specifically, as shown in FIG. 2, the information processing submodule includes a first buffer and a lesion target detection buffer, a lesion segmentation buffer, and a semantic scene detection buffer that are connected to the first buffer.

The first buffer is connected to the AI edge computation module to receive and temporarily store the message pushed by the AI edge computation module and parse a message header of the message. According to the message header, the lesion target detection message is temporarily stored in the lesion target detection buffer, the lesion segmentation message is temporarily stored in the lesion segmentation buffer, and the semantic scene detection message is temporarily stored in the semantic scene detection buffer, so as to be divided into the lesion target detection message packet, the lesion segmentation message packet, and the semantic scene detection message packet respectively.

That is, if the message header of the message pushed by the AI edge computation module is DET, the first buffer determines that the message is the lesion target detection message and stores the lesion target detection message in the lesion target detection buffer. If the message header of the message pushed by the AI edge computation module is SEG, the first buffer determines that the message is the lesion segmentation message and stores the lesion segmentation message in the lesion segmentation buffer. If the message header of the message pushed by the AI edge computation module is TXT, the first buffer determines that the message is the semantic scene detection message and stores the semantic scene detection message in the semantic scene detection buffer. Thus, the lesion target detection buffer temporarily stores a plurality of lesion target detection messages, that is, a lesion target detection message packet. The lesion segmentation buffer temporarily stores a plurality of lesion segmentation messages, that is, a lesion segmentation message packet. The semantic scene detection buffer temporarily stores a plurality of semantic scene detection messages, that is, a semantic scene detection message packet. Thus, the packet marking of pushed messages is implemented.

In the present invention, a validity period of the lesion target detection buffer, a validity period of the lesion segmentation buffer, and a validity period of the semantic scene detection buffer are set as 16.66 ms. It should be noted that 16.66 ms depends on an access frequency of the image pushing submodule. In the present invention, the image pushing submodule adopts an access frequency of 60 frames per second, such that 1000/60≈16.66. In actual use, the validity period is not limited thereto, and may vary according to the access frequency of the image pushing submodule.

Meanwhile, when receiving, the first buffer may receive the message pushed by the AI edge computation module through the Socker Client.

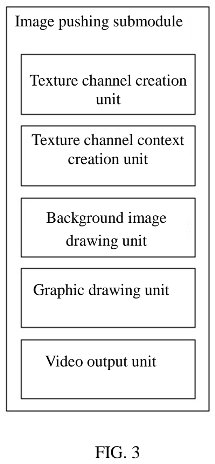

As shown in FIG. 3, in the present invention, the image pushing submodule includes a texture channel creation unit, a texture channel context creation unit, a background image drawing unit, a graphic drawing unit, and a video output unit.

The texture channel creation unit is configured to create and bind a lesion target detection texture channel, a lesion segmentation texture channel, and a semantic scene detection texture channel to three output ports respectively.

In the present invention, the texture channel creation unit may create a lesion target detection texture channel, a lesion segmentation texture channel, and a semantic scene detection texture channel by using GLFW software. Specifically, the glfwGetMonitorPos function may be used to select the video output port. The glfwGetMonitorPos function supports input of a serial number of video transmission. For example, the lesion target detection texture channel is bound to a number 1 output port, that is, getMonitors()[1] is input. By analogy, the lesion segmentation texture channel may be bound to a number 2 output port and the semantic scene detection texture channel may be bound to a number 3 output port.

The texture channel context creation unit is configured to create a lesion target detection texture channel context, a lesion segmentation texture channel context, and a semantic scene detection texture channel context for the three output ports respectively.

In the present invention, the glfwMakeContextCurrent function in GLFW software may be used to bind the three output ports to the OpenGL context, so as to obtain the lesion target detection texture channel context, the lesion segmentation texture channel context, and the semantic scene detection texture channel context.

The background image drawing unit is configured to draw background images in the lesion target detection texture channel, the lesion segmentation texture channel, and the semantic scene detection texture channel respectively.

In the present invention, since 60 frames are rendered circularly every second, when the frame buffer of each frame is initialized, the glClearColor function is used to set a clearing value. This function receives four parameters, corresponding to red, green, blue, and alpha (transparency) components. The default setting background of the present invention is white, that is, input parameters are 1.0, 1.0, 1.0, and 1.0. That is, glClearColor function is used and parameters 1.0, 1.0, 1.0, and 1.0 are input, that is, a white background image may be drawn in each texture channel. The present invention adopts the white background image, such that the background image does not affect the original video input signal during video overlaying.

The graphic drawing unit draws a lesion target graphic on the background image in the lesion target detection texture channel based on the lesion target detection message packet, draws a lesion segmentation graphic on the background image in the lesion segmentation texture channel based on the lesion segmentation message packet, and draws a semantic scene graphic on the background image in the semantic scene detection texture channel based on the semantic scene detection message packet.

Since the lesion target graphic is rectangular and the lesion segmentation graphic is polygonal, and drawing principles and methods are the same, the lesion target graph and the lesion segmentation graph will be introduced together below.

At first, the lesion target detection texture channel reads the lesion target detection message packet frame by frame from the lesion target detection buffer, and the lesion segmentation texture channel reads the lesion segmentation message packet frame by frame from the lesion segmentation buffer, obtains coordinates of each vertex in each message from the corresponding message packet, and converts, by using the glBufferData function, the vertex coordinates into vertex buffer objects that may be interpreted by a graphics processing unit (GPU). Then, the glBindBuffer method is invoked to bind the vertex buffer object that may be interpreted by the GPU to the corresponding lesion target detection texture channel context or lesion segmentation texture channel context. Finally, the glDrawArrays method is invoked to complete graphic drawing. The method may obtain vertex data from the corresponding context and draw the corresponding graphics on the background image of a current texture channel.

Specifically, the lesion target graphic is a rectangular box, and a format of vertex coordinates is [(x0min,y0min), (x0max,y0max)],[(x1min,y1min), (x1max,y1max)]...[(xn'min,yn'min), (xn'max,yn'max)]. Each "[]" represents a graphic object, xmin and ymin represent the upper left corner coordinates of each graphic, and xmax and ymax represent the lower right corner coordinates of the graphic.

The lesion segmentation graphic is a polygon, and a format of coordinate vertices is [(x0,y0), (x1,y1),....,(xn,yn)],......,[(x0,y0), (x1,y1),....,(xn,yn)]. Each "[]" represents a graphic object and stores the coordinates of each vertex in the graphic.

Since the semantic scene graphic is a character graphic, how to draw the character graphic in the semantic scene detection texture channel will be introduced herein separately. For the convenience of introduction, the present invention will use an example by assuming that the message body of the semantic scene detection message in the semantic scene detection buffer is "Pay attention to flushing the intestine", specific steps of drawing the corresponding character graphic are as follows:

At first, characters are traversed one by one, and a character texture set is searched according to the character values for corresponding matched texture object.

Secondly, a position of the character is computed. According to the length and position of the character, it is assumed that the position of a displayed character in the present invention is centered to a bottom of a 1920*1080 screen, the character size is 14 pixels, and illustrative characters include 7 characters. Thus, coordinates of a vertex position of a first character are: (896. 1060),(910, 1060). By analogy, if a character spacing is 2 pixels, coordinates of a vertex position of a second character are: (912, 1054),(926, 1054), and the remaining character computation solutions are based on such computation.

Then, the vertex coordinates of the characters are input one by one, and the vertex coordinates are converted, by using glBufferData function, into vertex buffer objects that may be interpreted by the GPU. The glBindBuffer method is invoked to bind the vertex buffer object that may be interpreted by the GPU to the corresponding semantic scene detection texture channel context.

Finally, the glDrawArrays method is invoked to complete character graphic drawing. The method may obtain vertex data from the corresponding context and draw the corresponding texture graphic content on the background image of a current texture channel.

It should be known that the character texture set exist as a fixed content, that is, every time the program is initialized, and specific creation steps of the character texture set include:

At first, the FreeType software library is used to load a font file and invoke FT_Set_Pixel_Sizes to set the required font pixel size (the font belongs to the conventional technical feature, and regarding the font size, the present invention uses the following formula to compute the font size, 10.5 pounds/72*96 dpi=14 pixels, that is, one pound equals 1/72 inch, and the present invention assumes display on a 96 dpi display).

Secondly, the character texture set is created. Specifically, since the graphic library used in the present invention is OpenGL, the characters in the font file is traversed, the glBindTexture function is invoked to obtain a texture object, a character pattern of each character is obtained through loading by FreeType and is onverted into bitmap information, and the bitmap is copied to the texture object. A bitmap includes a texture content, coordinates, a size, a baseline position, and a character width of each character.

Finally, a mapping set is created. In the present invention, key values are used to store data types, and keys are characters and the values are texture objects.

The video output unit is configured to form the background image and the lesion target graphic drawn thereon into the lesion target detection result video, form the background image and the lesion segmentation graphic drawn thereon into the lesion segmentation result video, and form the background image and the semantic scene graphic drawn thereon into the semantic scene detection result video, and output such videos to the video overlaying module through the three output ports respectively.

Specifically, a frame buffer refresh function may be invoked to continuously output corresponding graphics, to form the video output.

The video overlaying module is configured to overlay the lesion target detection result video, the lesion segmentation result video, and the semantic scene detection result video from the image pushing submodule and the video input signal from the video acquisition module and output an overlaid video to the display module.

Specifically, the video overlaying module includes four input terminals and one output terminal. The four input terminals are configured to receive the lesion target detection result video, the lesion segmentation result video, and the semantic scene detection result video that are from the video output unit and the video input signal from the video acquisition module. The video overlaying module overlays received videos. The output terminal is configured to output the overlaid video to the display module.

In the present invention, the video overlaying module may adopt a field programmable gate array (FPGA) video overlaying module. When the FPGA video overlaying module overlays the received videos, an overlaying sequence from the bottom to the top is that the video input signal from the video acquisition module, that is, an original video input frame→ the lesion target detection result video→the lesion segmentation result video→the semantic scene detection result video, and finally the overlaid and synthesized AI frame is obtained.

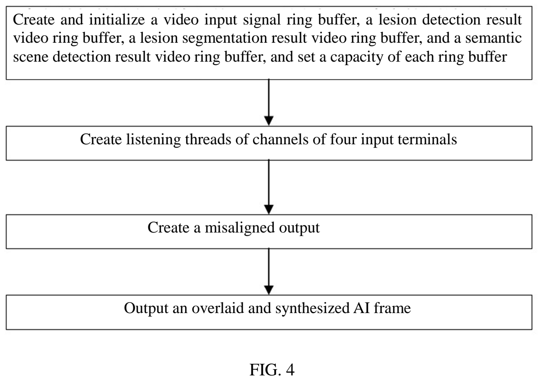

As shown in FIG. 4, specific steps of video overlaying of the FPGA video overlaying module are as follows:

1) A video input signal ring buffer, a lesion detection result video ring buffer, a lesion segmentation result video ring buffer, and a semantic scene detection result video ring buffer are created and initialized, and a capacity of each ring buffer is set.

Each ring buffer needs to implement interface logic of a memory chip: a DDR3 memory chip is connected to an external memory interface of the FPGA video overlaying module, and the DDR3 controller IP core and a memory interface generator (MIG) tool are used in the FPGA video overlaying module to generate the memory controller and interface. The MIG tool may be used to generate the memory controller and interface according to the model and parameters of the memory chip. For a capacity of the ring buffer, the present invention assumes receiving of signal data of 1920*1080 pixels and 24-bit RGB graphics (accounting for 3 bytes), and 3 frames are stored by default. The capacity of the ring buffer is as follows:

$$1920*1080*3(bytes)*3(frames)=18662400 \text{ bytes.}$$

2, Listening threads of four channels, that is, listening threads of input channels of four input terminals are created. In this way, when the input terminals receive the video input, the input terminals may be listened to and write the frame data into the corresponding ring buffer. When the capacity of the ring buffer is full, header frame data are popped while new frame data are inserted.

3, A misaligned output is created. Low delay output of the original image can be ensured through misaligned output, and stable marking information can be obtained.

The creation of the misaligned output specifically includes:
At first, when the video input signal ring buffer reaches a preset storage length for first time, first frame data are output without any processing.

In the present invention, the IP core of Xilinx HDMI 2.0 interface may be used to implement the HDMI 2.0 output interface. Specifically, the IP core of a double data rate (DDR) memory controller reads and provides video frame data from the DDR for a finite state machine (FSM). The FSM controls sequence and time of data reading and data sending to the HDMI interface according to an internal state. In addition, the FSM controls the generation of synchronization signals, including horizontal synchronization, vertical synchronization, and pixel enable signals. These synchronization signals determine a specific format and timing of video frames, and finally make the HDMI interface output images in the correct format and speed.

Secondly, after the output of the first frame data, subsequent new frame data continue to enter the video input signal ring buffer. In this case, the first frame data in the video input signal ring buffer and first frame data in the other three ring buffers are misaligned by time of a frame.

4, An overlaid and synthesized AI frame is output.

At first, output time of the first frame data of the video input signal occupied by the misaligned output is used for graphic overlaying on header first frame data of the lesion detection result video ring buffer, header first frame data of the lesion segmentation result video ring buffer, and header first frame data of the semantic scene detection result video ring buffer, and a synthesized AI graphic prompt signal is obtained (it should be noted that when this step is implemented, weather the graphic overlaying is completed within 1 frame time, that is, 1 second /60 frames≈16.66 ms, depends on a running speed of the FPGA chip. For example, a too low-end FPGA chip may not satisfy this performance, but the present invention uses the high-end FPGA chip to satisfy this performance).

In the present invention, the synthesized AI graphic prompt signal is obtained by the following steps: (1) the first frame data are taken from the lesion target detection result video ring buffer, the lesion segmentation result video ring buffer, and the semantic scene result video ring buffer, and the pixels are traversed in turn according to the size information. (2) Weather the obtained three pixel values are background colors (according to the context, the background colors are assumed to be white 255, 255, 255) is determined. (3) If the pixel values are all background colors, the pixel value of the AI graphic prompt signal at this position is set as the background color; if there are 1-3 non-background color values among the three pixel values, the pixel value of the AI graphic prompt signal at this position is set according to the priority order. The priority order is that 1. a frame data pixel value corresponding to the semantic scene result video→2. a frame data pixel value corresponding to the lesion segmentation result video→3. a frame data pixel value corresponding to the lesion detection result video. (4) The synthesized AI graphic prompt signal is finally obtained.

Then, the synthesized AI graphic prompt signal and the frame data of the video input signal are overlaid. Specifically, the frame data of the video input signal are read from the video input signal ring buffer, and pixel-by-pixel numerical determination is performed with the synthesized AI graphic prompt signal. If the numerical value is the same as the background color (herein, it is assumed to be white 255, 255, 255 according to the context), the pixels of the frame data of the video input signal are output. If the numerical value is different from the background color, the pixel value is set as the synthesized AI graphic prompt signal, and finally the overlaid and synthesized AI frame is obtained.

The display module is configured to display the overlaid video.

Preferably, an internal structure of the display module includes a video input terminal chip, a video output terminal chip, and an image input source switch button. The video acquisition module, the AI edge computation module, and the video overlaying module are all connected to the video input terminal chip of the display module. The image input source switch button is configured to determine whether a content displayed by the display module is from the video acquisition module, the AI edge computation module, or the video overlaying module.

In this way, the display module may perform display according to the display mode selected by a user. The user may control switch of the display mode through the image input source switch button. A display mode 1 is to directly display the original video input signal of the medical imaging device. A display mode 2 is to display the video signal that has marking information and is processed by the AI edge computation module and output by the marking layer output module and overlaid with the original video input signal. A display mode 3 is to output an operating system HDMI signal by the AI edge computation module. Thus, the present invention provides three display modes, such that the system has a multifunctional effect, and provides more convenience for medical personnel.

Finally it should be noted that the above examples are merely used to describe the technical solution of the present invention rather than limit the protection scope of the present invention. According to the idea of the present invention, those skilled in the art can modify or replace equivalently the technical solution of the present invention without departing from the essence and scope of the technical solution of the present invention.

## Claims

1. An intelligent medical display system, comprising a video acquisition module, an artificial intelligence (AI) edge computation module, a marking layer output module, a video overlaying module, and a display module, wherein

   the video acquisition module is configured to receive a video input signal from a medical imaging device and transmit the video input signal to the AI edge computation module;
   the AI edge computation module is configured to analyze the video input signal to obtain and push a lesion target detection message, a lesion segmentation message, and a semantic scene detection message to the marking layer output module;
   the marking layer output module comprises an information processing submodule and an image pushing submodule, the information processing submodule is configured for packet marking on the messages pushed by the AI edge computation module, and divide the messages into a lesion target detection message packet, a lesion segmentation message packet, and a semantic scene detection message packet, and the image pushing submodule is configured to mark a background image according to a packet marking result of the information processing submodule, to form and output a lesion target detection result video, a lesion segmentation result video, and a semantic scene detection result video to the video overlaying module;
   the video overlaying module is configured to overlay the lesion target detection result video, the lesion segmentation result video, and the semantic scene detection result video from the image pushing submodule and the video input signal from the video acquisition module and output an overlaid video to the display module; and
   the display module is configured to display the overlaid video.

2. The intelligent medical display system according to claim 1, **characterized in that** the information processing submodule comprises a first buffer and a lesion target detection buffer, a lesion segmentation buffer, and a semantic scene detection buffer that are connected to the first buffer; the first buffer is connected to the AI edge computation module to receive and temporarily store the message pushed by the AI edge computation module and parse a message header of the message; and according to the message header, the lesion target detection message is temporarily stored in the lesion target detection buffer, the lesion segmentation message is temporarily stored in the lesion segmentation buffer, and the semantic scene detection message is temporarily stored in the semantic scene detection buffer, so as to be divided into the lesion target detection message packet, the lesion segmentation message packet, and the semantic scene detection message packet respectively.

3. The intelligent medical display system according to claim 2, **characterized in that** the image pushing submodule comprises a texture channel creation unit, a texture channel context creation unit, a background image drawing unit, a graphic drawing unit, and a video output unit;

   the texture channel creation unit is configured to create and bind a lesion target detection texture channel, a lesion segmentation texture channel, and a semantic scene detection texture channel to three output ports respectively;
   the texture channel context creation unit is configured to create a lesion target detection texture channel context, a lesion segmentation texture channel context, and a semantic scene detection texture channel context for the three output ports respectively;
   the background image drawing unit is configured to draw background images in the lesion target detection texture channel, the lesion segmentation texture channel, and the semantic scene detection texture channel respectively;
   the graphic drawing unit draws a lesion target graphic on the background image in the lesion target detection texture channel based on the lesion target detection message packet, draws a lesion segmentation graphic on the background image in the lesion segmentation texture channel based on the lesion segmentation message packet, and draws a semantic scene graphic on the background image in the semantic scene detection texture channel based on the semantic scene detection message packet; and

the video output unit is configured to form the background image and the lesion target graphic drawn thereon into the lesion target detection result video, form the background image and the lesion segmentation graphic drawn thereon into the lesion segmentation result video, and form the background image and the semantic scene detection graphic drawn thereon into the semantic scene detection result video, and output such videos to the video overlaying module through the three output ports respectively.

4. The intelligent medical display system according to claim 3, **characterized in that** the video overlaying module comprises four input terminals and one output terminal, the four input terminals are configured to receive the lesion target detection result video, the lesion segmentation result video, and the semantic scene detection result video that are from the video output unit and the video input signal from the video acquisition module, and the output terminal is configured to output the overlaid video to the display module.

5. The intelligent medical display system according to claim 4, **characterized in that** the video overlaying module performs video overlaying through the following steps:

1) creating and initializing a video input signal ring buffer, a lesion detection result video ring buffer, a lesion segmentation result video ring buffer, and a semantic scene detection result video ring buffer, and setting a capacity of each ring buffer;
2) creating four listening threads of input channels of the input terminals, wherein when the input terminals receive a video input, the input terminals write frame data to corresponding ring buffers respectively, and when the ring buffer is full, header frame data are popped while new frame data are inserted;
3) creating a misaligned output, that is, directly outputting, when the video input signal ring buffer reaches a preset storage length for first time, first frame data without any processing, wherein the first frame data in the video input signal ring buffer and first frame data in the other three ring buffers are misaligned by time of a frame; and
4) outputting an overlaid and synthesized AI frame.

6. The intelligent medical display system according to claim 5, **characterized in that** step 4) specifically comprises:

4.1) using output time of the first frame data of the video input signal occupied by the misaligned output for graphic overlaying on header first frame data of the lesion detection result video ring buffer, header first frame data of the lesion segmentation result video ring buffer, and header first frame data of the semantic scene detection result video ring buffer, and obtaining a synthesized AI graphic prompt signal; and
4.2) overlaying the synthesized AI graphic prompt signal and the frame data of the video input signal and obtaining the overlaid and synthesized AI frame.

7. The intelligent medical display system according to claim 1, **characterized in that** the AI edge computation module comprises a lesion target detection submodule, a lesion segmentation submodule, and a semantic scene detection submodule, the lesion target detection submodule is configured to recognize the video input signal frame by frame to obtain a lesion detection object target, and convert the lesion detection object target into a message to obtain the lesion target detection message, the lesion segmentation submodule is configured to recognize the video input signal frame by frame to obtain a lesion segmentation object target, and convert the lesion segmentation object target into a message to obtain the lesion segmentation message, and the semantic scene detection submodule is configured to recognize the video input signal frame by frame to obtain a semantic scene detection result, and convert the semantic scene detection result into a message to obtain the semantic scene detection message.

8. The intelligent medical display system according to claim 7, **characterized in that** the lesion target detection message comprises a message header and a message body, the message header is DET, the message body comprises coordinates of a plurality of lesion detection object targets, and a plurality of coordinates are separated by a symbol "|"; the lesion segmentation message comprises a message header and a message body, the message header is SEG, the message body is a set of point coordinates of a plurality of lesion segmentation object targets, and a plurality of point coordinate sets are separated by a symbol |; and the semantic scene detection message comprises a message header and a message body, the message header is TXT, the message body comprises prompt words corresponding to a plurality of semantic scenes, and a plurality of prompt words are separated by a symbol |.

9. The intelligent medical display system according to claim 1, **characterized in that** the display module has an image input source switch button, the video acquisition module, the AI edge computation module, and the video overlaying module are all connected to the display module, and the image input source switch button is configured to determine whether a content displayed by the display module is from the video acquisition module, the AI edge computation module, or the video overlaying module.

FIG. 1

```
┌─────────────────────────────────────────────┐
│          AI edge computation module          │
└─────────────────────────────────────────────┘
                      │
                      ▼
┌─────────────────────────────────────────────┐
│                 First buffer                 │
└─────────────────────────────────────────────┘
                      │
    ┌─────────────────┼─────────────────┐
    ▼                 ▼                 ▼
┌──────────┐    ┌──────────┐    ┌──────────┐
│ Lesion   │    │ Lesion   │    │ Semantic │
│ target   │    │ segment- │    │ scene    │
│ detection│    │ ation    │    │ detection│
│ buffer   │    │ buffer   │    │ buffer   │
└──────────┘    └──────────┘    └──────────┘
    │                 │                 │
    ▼                 ▼                 ▼
┌─────────────────────────────────────────────┐
│            Image pushing submodule           │
└─────────────────────────────────────────────┘
    │                 │                 │
    ▼                 ▼                 ▼
┌──────────┐    ┌──────────┐    ┌──────────┐
│ Lesion   │    │ Lesion   │    │ Semantic │
│ target   │    │ segment- │    │ scene    │
│ detection│    │ ation    │    │ detection│
│ result   │    │ result   │    │ result   │
│ video    │    │ video    │    │ video    │
└──────────┘    └──────────┘    └──────────┘
```

FIG. 2

Image pushing submodule

Texture channel creation unit

Texture channel context creation unit

Background image drawing unit

Graphic drawing unit

Video output unit

FIG. 3

Create and initialize a video input signal ring buffer, a lesion detection result video ring buffer, a lesion segmentation result video ring buffer, and a semantic scene detection result video ring buffer, and set a capacity of each ring buffer

↓

Create listening threads of channels of four input terminals

↓

Create a misaligned output

↓

Output an overlaid and synthesized AI frame

FIG. 4

## INTERNATIONAL SEARCH REPORT

| | International application No. |
|---|---|
| | **PCT/CN2024/090491** |

| A. | CLASSIFICATION OF SUBJECT MATTER |
|---|---|

G16H30/20(2018.01)i; G16H30/40(2018.01)i; G06T7/00(2017.01)i; G06T7/10(2017.01)i; H04N5/265(2006.01)i

According to International Patent Classification (IPC) or to both national classification and IPC

| B. | FIELDS SEARCHED |
|---|---|

Minimum documentation searched (classification system followed by classification symbols)

IPC:G16H30/-; G06T7/-; H04N5/-

Documentation searched other than minimum documentation to the extent that such documents are included in the fields searched

Electronic data base consulted during the international search (name of data base and, where practicable, search terms used)

CNABS, CNTXT, CNKI, ENTXTC, VEN, ENTXT, IEEE: 视频, 采集, 边缘计算, 标记, 病变, 分析, 叠加, 显示, 图像, 缓冲, 切割, 场景, 纹理, 背景, 输入, 输出, 报文, 天津御锦人工智能医疗, video, collect, edge computing, label, pathological change, analyze, merge, display, image, buffer, cut, background, input, output, message, TIANJIN YUJIN ARTIFICIAL INTELLIGENCE MEDICAL

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 116721740 A (TIANJIN YUJIN ARTIFICIAL INTELLIGENCE MEDICAL TECHNOLOGY CO., LTD.) 08 September 2023 (2023-09-08) <br> claims 1-9 | 1-9 |
| Y | CN 114724682 A (CHENGDU YURUI INNOVATION TECHNOLOGY CO., LTD.) 08 July 2022 (2022-07-08) <br> description, paragraphs 5, 19, and 34, and figures 2-5 | 1-2, 7-9 |
| Y | CN 113542842 A (STATE GRID INFORMATION & TELECOMMUNICATION CO., LTD.) 22 October 2021 (2021-10-22) <br> description, paragraph 45 | 1-2, 7-9 |
| A | CN 112132772 A (HANGZHOU DEEPINFORAMTICS++ TECHNOLOGY CO., LTD.) 25 December 2020 (2020-12-25) <br> entire document | 1-9 |

☑ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **26 June 2024** | **03 July 2024** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/090491** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 113645416 A (SHENZHEN MINDRAY ANIMAL MEDICAL TECHNOLOGY CO., LTD.) 12 November 2021 (2021-11-12) entire document | 1-9 |
| A | CN 114496173 A (BEIJING AEROSPACE CHANGFENG CO., LTD.) 13 May 2022 (2022-05-13) entire document | 1-9 |
| A | WO 2023279199 A1 (A.I. VALI INC.) 12 January 2023 (2023-01-12) entire document | 1-9 |

**INTERNATIONAL SEARCH REPORT**
Information on patent family members

International application No.

**PCT/CN2024/090491**

| Patent document cited in search report | | | Publication date (day/month/year) | Patent family member(s) | | | Publication date (day/month/year) |
|---|---|---|---|---|---|---|---|
| CN | 116721740 | A | 08 September 2023 | None | | | |
| CN | 114724682 | A | 08 July 2022 | CN | 114724682 | B | 16 August 2022 |
| CN | 113542842 | A | 22 October 2021 | None | | | |
| CN | 112132772 | A | 25 December 2020 | CN | 112132772 | B | 23 February 2024 |
| CN | 113645416 | A | 12 November 2021 | CN | 113645416 | B | 11 February 2022 |
| CN | 114496173 | A | 13 May 2022 | None | | | |
| WO | 2023279199 | A1 | 12 January 2023 | EP | 4367683 | A1 | 15 May 2024 |
| | | | | CA | 3223508 | A1 | 12 January 2023 |
| | | | | IN | 202417005753 | A | 15 March 2024 |
| | | | | CN | 117836870 | A | 05 April 2024 |

Form PCT/ISA/210 (patent family annex) (July 2022)

**EP 4 712 095 A1**

**Patent documents cited in the description**

- CN 202310536781